# EUROPEAN PATENT SPECIFICATION

(11) **EP 0 608 336 B1**
(45) Date of publication and mention of the grant of the patent: **20.05.1998**
(21) Application number: 92921974.9
(22) Date of filing: 09.10.1992
(51) Int. Cl.: C07D 487/08, A61K 31/40

(54) **AZABICYCLO ARYLACETYLENE AND ARYLENYNE OXIMES AS CHOLINERGIC AGENTS AND METHODS OF TREATMENT**
AZABICYCLOARYLACETYLEN- UND ARYLENYNOXINE ALS CHOLINERGISCHE MITTEL UND VERFAHREN ZUR BEHANDLUNG
OXIMES D'ARYLENYNE ET D'ARYLACETYLENE AZABICYCLO UTILISES COMME AGENTS CHOLINERGIQUES, ET PROCEDES DE TRAITEMENT

(30) Priority: 16.10.1991 US 778412
(43) Date of publication of application: 03.08.1994
(73) Proprietor: WARNER-LAMBERT COMPANY, Ann Arbor, Michigan 48105 (US)
(72) Inventor: LAUFFER, David, Jeffrey, Saline, MI 48176 (US); MOOS, Walter, Hamilton, Moos, Oakland, CA 94611 (US); PAVIA, Michael, Raymond, Newton, MA 81259 (US); TECLE, Haile, Ypsilanti, MI 48197 (US); THOMAS, Anthony, Jerome, Ann Arbor, MI 48104 (US)
(74) Representative: Mansmann, Ivo
(86) International application number: PCT/US92/08642
(87) International publication number: WO 93/08192

(56) References cited:
- EP-A- 0 445 731
- US-A- 4 937 239

## Description

### FIELD OF INVENTION

The present invention is a class of oximes which are muscarinic agonists, rendering them useful as pharmaceutical agents. More specifically, the compounds are arylacetylene and arylenyne azabicyclic oximes.

### BACKGROUND

Disorders of cognition are generally characterized by symptoms of forgetfulness, confusion, memory loss, attentional deficits, and/or, in some cases, affective disturbances. These symptoms may arise as a result of the general aging process and/or from organic brain disease, cerebrovascular disease, head injury, or developmental or genetic defects.

The general decrease in cognitive function which accompanies the aging process is well accepted. The same phenomenon has been observed and documented in many lower mammals, including those routinely employed in pharmacological testing programs for screening and predicting usefulness for particular drugs in higher animals, including humans.

Although disorders of cognition often accompany the general aging process, presenile and senile primary degenerative dementia are the most common accepted causes of mental deterioration in the elderly. It has been estimated that at least ten percent of persons over 60 years of age will eventually suffer severe mental deterioration. A much larger number will experience cognitive decline of sufficient severity to impede their activities.

Many of the symptoms of cognitive disorders, especially impaired memory, are associated with decreased acetylcholine synthesis and the impairment of cholinoreceptive neurons. In the hippocampus and cerebral cortex of patients suffering from primary degenerative dementia, for example, the level of the enzyme choline acetyltransferase (CAT) can be reduced by as much as 90%. (See Davies, et al, The Lancet, 1976; (Vol. 2):1403; Perry, et al, J. Neurol. Sci., 1977;34:247-265; and White, et al, The Lancet, 1977; (Vol. 1):668-670).

Since CAT catalyzes the synthesis of acetylcholine from its precursors choline and acetyl coenzyme A, the loss of CAT reflects the loss of cholinergic, or acetylcholine-releasing, nerve endings in the hippocampus and cerebral cortex. There is abundant evidence that cholinergic terminals in the hippocampus are critically important for memory formation.

The cholinergic hypothesis suggests that drugs which restore acetylcholine levels or which mimic the action of acetylcholine (i.e., are cholinomimetic) are effective in correcting this deficit in neurotransmitter chemical and provide treatment of the memory impairment symptom of cerebral insufficiency. Considerable biochemical, pharmacological, and electrophysiological evidence supports the hypothesis that deficits in the cholinergic system underlie geriatric cognitive dysfunction. (See C. Peterson and G. E. Gibson, Neurobiol. Aging, 1983;4:25-30). Aged humans and nonhuman primates with decreased cognition show improved memory when they are treated, for example, with acetylcholinesterase inhibitors such as physostigmine. These agents increase the available supply of synaptic acetylcholine by inhibiting its hydrolysis.

Aminopyridines such as 3,4-diaminopyridine ameliorate age-related cognitive deficits by increasing the release of acetylcholine from presynaptic nerve terminals, thus increasing synaptic acetylcholine. (See H. P. Davis, et al, Exp. Aging Res., 1983;9:211-214).

It has been known for some time that the natural alkaloid, muscarine, has the ability to act relatively selectively at autonomic effector cells to produce qualitatively the same effects as acetylcholine. Two alkaloids, pilocarpine and arecoline (the methyl ester of 1,2,5,6-tetrahydro-1-methyl-3-pyridinecarboxylic acid), have the same principal sites of action as muscarine and acetylcholine and are thus classified as having "muscarinic" action. Although these naturally occurring alkaloids are of great value as pharmacological tools, present clinical use is largely restricted to the use of pilocarpine as miotic agent.

Recently it has been demonstrated that arecoline is effective in ameliorating some of the symptoms of cognitive disorders in patients clinically diagnosed as having presenile primary degenerative dementia. Significant improvement was observed in a test of picture recognition after administration of arecoline to patients in a double-blind study. (See Christie, et al, Brit. J. Psychiatry, 1981;138:46-50).

The use of cholinomimetic agents in multiple clinical trials has documented both the potential therapeutic utility of cholinergic agents and the high incidence of unwanted side effects (see E. Hollander, et al, Brit. Med. Bull. 1986;42:97-100). Many of these unwanted side effects result from the nonselective stimulation of cholinergic receptors (of the muscarinic type) located throughout the body. Muscarinic receptors have been classified pharmacologically and at the molecular level into several subtypes (see T. Bonner, Trends Pharmacol. Sci. 1989 (Suppl. on Subtypes of Muscarinic Receptors IV):11-15). The receptors responsible for the central cognition-enhancing effects of muscarinic cholinomimetic agents are generally defined as M₁ (pharmacological definition) or ml (molecular definition). Activation of peripheral M₂ and M₃ (or m2 and m3) receptors is thought to be responsible for the unwanted side effects of the currently available muscarinic agents (e.g., sweating, diarrhea, cramps, excessive salivation, etc). Thus, selective M₁ (or m1) muscarinic agonists hold the best promise for selective improvement of cognitive function without the occurrence of unwanted side effects.

Compounds of formula (i) are disclosed in EP-0445731A as centrally active muscarinic agents for use as analgesics and for the treatment of a number of conditions of cerebral insufficiency characterized by decreased cerebral acetylcholine production or release. In particular, compounds (ii) and (iii) are specifically described as muscarinic agonists, although no biological data are presented to support this claim. In general, the compounds described in EP-0445731A are predicted to possess agonist activity at muscarinic receptors from the relative ability of the compounds to displace radiolabeled muscarinic antagonists and agonists from muscarinic receptors. However, no data are given in EP-0445731A to suggest that the compounds will possess selectivity for a given muscarinic subtype, or that they will be free of the usual muscarinic side effects mentioned above.

We have now discovered that compounds related to (ii), where the aromatic group is properly substituted, and compounds related to (iii), where an additional aromatic group (substituted or unsubstituted) has been added at the terminus of the oxime side chain, and alkyl substituents are added on the olefinic carbons of the oxime side chain, are some of the most potent and ml-selective muscarinic agonists known. This subtype selectivity has been documented by competitive binding assays in cloned cell lines that express only one of the muscarinic receptor subtypes, and by measuring second-messenger responses in the same cell lines. These data indicate that the compounds disclosed in this application possess greater selectivity for m1 receptors than the compounds disclosed in EP-0445731A; but even more important are the findings from the second-messenger assays, which document that the novel compounds now disclosed are potent and selective activators of ml receptors, with minimal activity at the other muscarinic subtype receptors. This novel profile of activity distinguishes these compounds from those disclosed in EP-0455731A.

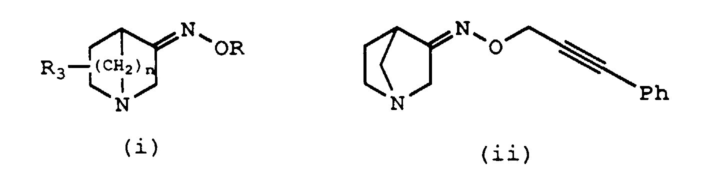

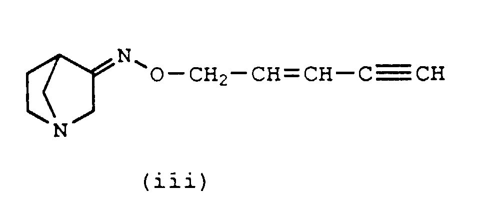

### SUMMARY OF THE INVENTION

The present invention is directed to compounds of the following general Formula I and pharmaceutically acceptable salts thereof, 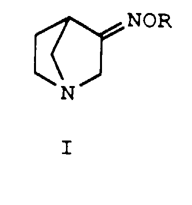 wherein R is selected from the group 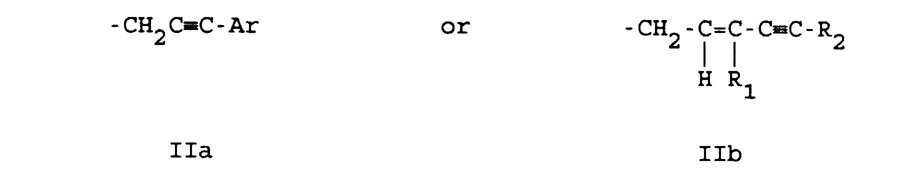 wherein Ar is an aromatic group selected from phenoxy, phenyl, 2- or 3-thienyl, 2- or 3-furanyl, 2- or 3-pyrrolyl, 2-, 3-, or 4-pyridinyl wherein the aromatic group is substituted with 1 or 2 substituents selected from straight or branched alkyl having from 1 to 4 carbon atoms, straight or branched alkoxy having from 1 to 4 carbon atoms, chlorine, bromine, fluorine, trifluoromethyl, nitro, hydroxy, trifluoromethoxy, amino, amino substituted with 1 or 2 straight or branched lower alkyl groups having from 1 to 4 carbon atoms;
R₂ is hydrogen or Ar as defined above and the corresponding unsubstituted aromatic groups;
R₁ is alkyl of 1 to 4 carbon atoms, preferably methyl.

The compounds of the present invention may exist in either of two Z and E isomeric forms of the oxime, see below, and enantiomeric forms. The present invention includes both forms of the compounds as well as mixtures of the Z and E forms. Illustratively Formulas III and IV depict the Z and E forms of the compounds of Formula I. Moreover, in those compounds in which there is a double bond in a carbon chain, both the Z and E forms of the olefins are included in the present invention.

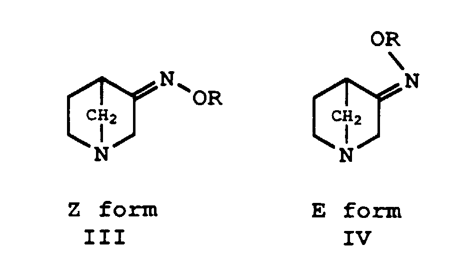

Pharmaceutical compositions comprising compounds of Formula I are also included in the present invention as well as the use of the compounds for the preparation of pharmaceuticals useful as analgesics and in the treatment of cognitive decline.

### DETAILED DESCRIPTION OF THE INVENTION

In the compounds depicted by general Formula I, the various substituents are further described as follows. Illustrative examples of straight or branched lower alkyl having from 1 to 4 carbon atoms including methyl, ethyl, n-propyl, isopropyl, n-butyl, or tert-butyl.

Illustrative of lower alkoxy groups having from 1 to 4 carbon atoms are methoxy, ethoxy, and n-propoxy.

Pharmaceutically acceptable acid addition salts of the compounds of Formula I are illustratively hydrochloric, sulfuric, phosphoric, acetic, benzoic, citric, malonic, salicylic, malic, fumaric, oxalic, succinic, tartaric, lactic, gluconic, ascorbic, maleic, aspartic, benzenesulfonic, methane and ethanesulfonic, hydroxymethane- and hydroxyethanesulfonic. (See, for example, "Pharmaceutical Salts," J. Pharm. Sci. 1977;66(1):1-19).

Preferred compounds of the present invention are those of Formula III wherein R is as defined above. The most preferred compounds of this invention are those of Formula III wherein R is of the Formula IIa or IIb in which R₁ is methyl and R₂ is Ar as defined above and the corresponding unsubstituted aromatic groups.

Illustrative of the most preferred hydrocarbon groups which R may represent are the following: -CH₂C≡C-Ar and 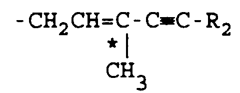 wherein * means the stereoconfiguration may be E or Z.

Most preferred Ar group is phenyl substituted with one substituent selected from straight alkyl from 1 to 4 carbons, straight alkoxy having from 1 to 4 carbon atoms, chlorine, bromine, trifluoromethyl, or trifluoromethoxy.

Most preferred R₂ group is hydrogen, phenyl or phenyl substituted with one substituent selected from straight alkyl from 1 to 4 carbons, straight alkoxy having from 1 to 4 carbon atoms, chlorine, bromine, trifluoromethyl, or trifluoromethoxy.

Particularly valuable are the following compounds:
Z-(±)-1-Azabicyclo[2.2.1]heptan-3-one, O-(3-(2'-methoxyphenyl)-2-propynyl)oxime;
Z-(+)-1-Azabicyclo[2.2.1]heptan-3-one, O-(3-(2'-methoxyphenyl)-2-propynyl)oxime;
Z-(-)-1-Azabicyclo[2.2.1]heptan-3-one, O-(3-(2'-methoxyphenyl)-2-propynyl)oxime;
Z-(±)-1-Azabicyclo[2.2.1]heptan-3-one, O-(3-(4'-methoxyphenyl)-2-propynyl)oxime;
Z-(+)-1-Azabicyclo[2.2.1]heptan-3-one, O-(3-(4'-methoxyphenyl)-2-propynyl)oxime;
Z-(-)-1-Azabicyclo[2.2.1]heptan-3-one, O-(3-(4'-methoxyphenyl)-2-propynyl)oxime;
Z-(±)-1-Azabicyclo[2.2.1]heptan-3-one, O-(3-(3'-methoxyphenyl)-2-propynyl)oxime;
Z-(+)-1-Azabicyclo[2.2.1]heptan-3-one, O-(3-(3'-methoxyphenyl)-2-propynyl)oxime;
Z-(-)-1-Azabicyclo[2.2.1]heptan-3-one, O-(3-(3'-methoxyphenyl)-2-propynyl)oxime;
Z-(±)-1-Azabicyclo[2.2.1]heptan-3-one, O-(3-(2'-chlorophenyl)-2-propynyl)oxime;
Z-(+)-1-Azabicyclo[2.2.1]heptan-3-one, O-(3-(2'-chlorophenyl)-2-propynyl)oxime;
Z-(-)-1-Azabicyclo[2.2.1]heptan-3-one, O-(3-(2'-chlorophenyl)-2-propynyl)oxime;
Z-(±)-1-Azabicyclo[2.2.1]heptan-3-one, O-(3-(3'-chlorophenyl)-2-propynyl)oxime;
Z-(+)-1-Azabicyclo[2.2.1]heptan-3-one, O-(3-(3'-chlorophenyl)-2-propynyl)oxime;
Z-(-)-1-Azabicyclo[2.2.1]heptan-3-one, O-(3-(3'-chlorophenyl)-2-propynyl)oxime;
Z-(±)-1-Azabicyclo[2.2.1]heptan-3-one, O-(3-(4'-chlorophenyl)-2-propynyl)oxime;
Z-(+)-1-Azabicyclo[2.2.1]heptan-3-one, O-(3-(4'-chlorophenyl)-2-propynyl)oxime;
Z-(-)-1-Azabicyclo[2.2.1]heptan-3-one, O-(3-(4'-chlorophenyl)-2-propynyl)oxime;
Z-(±)-1-Azabicyclo[2.2.1]heptan-3-one, O-(3-(2'-fluorophenyl)-2-propynyl)oxime;
Z-(+)-1-Azabicyclo[2.2.1]heptan-3-one, O-(3-(2'-fluorophenyl)-2-propynyl)oxime;
Z-(-)-1-Azabicyclo[2.2.1]heptan-3-one, O-(3-(2'-fluorophenyl)-2-propynyl)oxime;
Z-(±)-1-Azabicyclo[2.2.1]heptan-3-one, O-(3-(3'-fluorophenyl)-2-propynyl)oxime;
Z-(+)-1-Azabicyclo[2.2.1]heptan-3-one, O-(3-(3'-fluorophenyl)-2-propynyl)oxime;
Z-(-)-1-Azabicyclo[2.2.1]heptan-3-one, O-(3-(3'-fluorophenyl)-2-propynyl)oxime;
Z-(±)-1-Azabicyclo[2.2.1]heptan-3-one, O-(3-(4'-fluorophenyl)-2-propynyl)oxime;
Z-(+)-1-Azabicyclo[2.2.1]heptan-3-one, O-(3-(4'-fluorophenyl)-2-propynyl)oxime;
Z-(-)-1-Azabicyclo[2.2.1]heptan-3-one, O-(3-(4'-fluorophenyl)-2-propynyl)oxime;
Z-(±)-1-Azabicyclo[2.2.1]heptan-3-one, O-(Z-3-methyl-5-phenyl-2-penten-4-ynyl) oxime;
Z-(+)-1-Azabicyclo[2.2.1]heptan-3-one, O-(Z-3-methyl-5-phenyl-2-penten-4-ynyl)oxime;
Z-(-)-1-Azabicyclo[2.2.1]heptan-3-one, O-(Z-3-methyl-5-phenyl-2-penten-4-ynyl)oxime;
Z-(±)-1-Azabicyclo[2.2.1]heptan-3-one, O-(E-3-methyl-5-phenyl-2-penten-4-ynyl)oxime;
Z-(+)-1-Azabicyclo[2.2.1]heptan-3-one, O-(E-3-methyl-5-phenyl-2-penten-4-ynyl)oxime;
Z-(-)-1-Azabicyclo[2.2.1]heptan-3-one, O-(E-3-methyl-5-phenyl-2-penten-4-ynyl)oxime;
E-(±)-1-Azabicyclo-[2.2.1]heptan-3-one, O-(Z-3-methyl-5-phenyl-2-penten-4-ynyl)oxime;
E-(+)-1-Azabicyclo-[2.2.1]heptan-3-one, O-(Z-3-methyl-5-phenyl-2-penten-4-ynyl)oxime;
E-(-)-1-Azabicyclo-[2.2.1]heptan-3-one, O-(Z-3-methyl-5-phenyl-2-penten-4-ynyl)oxime;
E-(±)-1-Azabicyclo-[2.2.1]heptan-3-one, O-(E-3-methyl-5-phenyl-2-penten-4-ynyl)oxime;
E-(+)-1-Azabicyclo-[2.2.1]heptan-3-one, O-(E-3-methyl-5-phenyl-2-penten-4-ynyl)oxime;
E-(-)-1-Azabicyclo-[2.2.1]heptan-3-one, O-(E-3-methyl-5-phenyl-2-penten-4-ynyl)oxime;
Z-(±)-1-Azabicyclo-[2.2.1]heptan-3-one, O-(Z-3-methyl-2-penten-4-ynyl)oxime;
Z-(+)-1-Azabicyclo-[2.2.1]heptan-3-one, O-(Z-3-methyl-2-penten-4-ynyl)oxime;
Z-(-)-1-Azabicyclo-[2.2.1]heptan-3-one, O-(Z-3-methyl-2-penten-4-ynyl)oxime;
Z-(±)-1-Azabicyclo-[2.2.1]heptan-3-one, O-(E-3-methyl-2-penten-4-ynyl)oxime;
Z-(+)-1-Azabicyclo-[2.2.1]heptan-3-one, O-(E-3-methyl-2-penten-4-ynyl)oxime;
Z-(-)-1-Azabicyclo-[2.2.1]heptan-3-one, O-(E-3-methyl-2-penten-4-ynyl)oxime;
E-(±)-1-Azabicyclo-[2.2.1]heptan-3-one, O-(Z-3-methyl-2-penten-4-ynyl)oxime;
E-(+)-1-Azabicyclo-[2.2.1]heptan-3-one, O-(Z-3-methyl-2-penten-4-ynyl)oxime;
E-(-)-1-Azabicyclo-[2.2.1]heptan-3-one, O-(Z-3-methyl-2-penten-4-ynyl)oxime;
E-(±)-1-Azabicyclo-[2.2.1]heptan-3-one, O-(E-3-methyl-2-penten-4-ynyl)oxime;
E-(+)-1-Azabicyclo-[2.2.1]heptan-3-one, O-(E-3-methyl-2-penten-4-ynyl)oxime;and
E-(-)-1-Azabicyclo-[2.2.1]heptan-3-one, O-(E-3-methyl-2-penten-4-ynyl)oxime.

In addition to the novel compounds of the present invention as represented by Formula I above, the present invention provides novel pharmaceutical preparations.

The present invention provides pharmaceutical compositions useful as analgesic agents comprising an analgesically effective amount of a compound as defined above in combination with a pharmaceutically acceptable carrier. In another aspect, the present invention provides the use of the compounds for the preparation of pharmaceuticals useful for alleviating pain in a mammal.

In another aspect, the present invention provides pharmaceutical compositions for treating the symptoms of senile cognitive decline comprising a cholinergically effective amount of a compound as defined above in combination with a pharmaceutically acceptable carrier. In yet another aspect, the present invention provides the use of the compounds for the preparation of pharmaceuticals useful for treating the symptoms of senile cognitive decline in the elderly characterized by decreased cerebral acetylcholine production or release.

The compounds of Formula I are prepared by reacting a ketone of the following Formula V with a hydroxylamine (as the free base or salt) of the formula NH₂OR wherein R has the meanings defined in Formula I as depicted below:

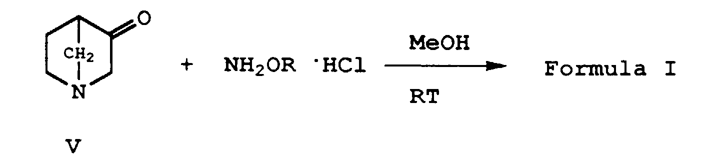

In the above depicted reaction, typically methanol is used as the solvent and the reaction is carried out at room temperature.

The salts of the final products are prepared by contacting the free base form of the compounds of this invention with a sufficient amount of the desired acid to produce the salt in the conventional manner. The free base forms may be regenerated, if desired, by treating the salt form with a base. For example, dilute aqueous solutions of such bases as sodium hydroxide, potassium carbonate, ammonia, and sodium bicarbonate may be utilized for this purpose.

The free base forms of the compounds of this invention differ somewhat from their respective salt forms in such physical properties as melting point and solubility in polar solvents, but the salts are otherwise equivalent to their respective free acid or base forms for the purposes of the invention.

The starting material represented by Formula V is known in the art. See, for example, J. Saunders, et al, J. Chem. Soc., Chem. Comm. 1988:1618 and R. J. Snow and L. J. Street, Tet. Lett. 1989;30:5795. Also, the hydroxylamines represented by NH₂OR are commercially available or may be prepared by procedures generally known in the art. For example, the hydroxylamines can be prepared by the Mitsunobu reaction by reacting an N-hydroxyphthalimide with an appropriate alcohol and hydrolyzing the resultant intermediate using methylhydrazine. Similarly, the N-hydroxyphthalimide can be reacted with an appropriate aliphatic or alicyclic bromide. The examples and preparations set forth below illustrate the synthesis of the compound of this invention.

### General Procedure for the Preparation of Oximes of Formula I

### 1-Azabicyclo[2.2.1] hept-3-one was prepared according to J. Chem. Soc., Chem. Comm. 1988:1618.

A solution of 1-azabicyclo[2.2.1]hept-3-one (2 g, 18 mmol) and the substituted hydroxylamine (hydrochloride salt) (H₂NOR·HCl; 18 mmol) in 10 mL methanol is stirred at room temperature for 18 hours. The reaction mixture is concentrated in vacuo. The residue is partitioned between CH₂Cl₂ and saturated potassium carbonate. The organic phase is dried over anhydrous sodium sulfate and evaporated in vacuo. The residue is converted to its oxalate salt and recrystallized from ethanol to afford the desired oxime as a mixture of E and Z oximes. The two geometric isomers can be separated by column chromatography on silica gel (CH₂Cl₂:MeOH; 9:1). Using this procedure, the following compounds were prepared:

### EXAMPLE 1

### Z-(±)-1-Azabicyclo[2.2.1]heptan-3-one, O-(3-(2'-methoxyphenyl)-2-propynyl)oxime oxalate, mp 155-157°C.

### EXAMPLE 2

### E-(±)-1-Azabicyclo[2.2.1]heptan-3-one, O-(3-(2'-methoxyphenyl)-2-propynyl)oxime oxalate, mp 132-133°C.

### EXAMPLE 3

### Z-(±)-1-Azabicyclo[2.2.1]heptan-3-one, O-(3-(3'-methoxyphenyl)-2-propynyl)oxime oxalate, mp 126-127°C.

### EXAMPLE 4

### E-(±)-1-Azabicyclo[2.2.1]heptan-3-one, O-(3-(3'-methoxyphenyl)-2-propynyl)oxime oxalate, mp 80-82°C.

### EXAMPLE 5

### Z-(±)-1-Azabicyclo[2.2.1]heptan-3-one, O-(3-(4'-methoxyphenyl)-2-propynyl)oxime oxalate, mp 158-159°C.

### EXAMPLE 6

### E-(±)-1-Azabicyclo[2.2.1]heptan-3-one, O-(3-(4'-methoxyphenyl)-2-propynyl)oxime oxalate, mp 147-148°C.

### EXAMPLE 7

### Z-(±)-1-Azabicyclo[2.2.1]heptan-3-one, O-(3-(2'-chlorophenyl)-2-propynyl)oxime oxalate, mp 143-145°C.

### EXAMPLE 8

### E-(±)-1-Azabicyclo[2.2.1]heptan-3-one, O-(3-(2'-chlorophenyl)-2-propynyl)oxime oxalate, mp 102-104°C.

### EXAMPLE 9

### Z-(±)-1-Azabicyclo[2.2.1]heptan-3-one, O-(3-(3'-chlorophenyl)-2-propynyl)oxime oxalate, mp 140-142°C.

### EXAMPLE 10

### E-(±)-1-Azabicyclo[2.2.1]heptan-3-one, O-(3-(3'-chlorophenyl)-2-propynyl)oxime oxalate, mp 114-116°C.

### EXAMPLE 11

### Z-(±)-1-Azabicyclo[2.2.1]heptan-3-one,O-(3-(4'-chlorophenyl)-2-propynyl)oxime oxalate, mp 163-164°C.

### EXAMPLE 12

### Z-(±)-1-Azabicyclo[2.2.1]heptan-3-one, O-(3-(4'-fluorophenyl)-2-propynyl)oxime oxalate, mp 144-145°C.

### EXAMPLE 13

### Z-(±)-1-Azabicyclo[2.2.1]heptan-3-one. O-(Z-3-methyl-5-phenyl-2-penten-4-ynyl)oxime oxalate, mp 137-141°C.

### EXAMPLE 14

### E-(±)-1-Azabicyclo[2.2.1]heptan-3-one, O-(Z-3-methyl-5-phenyl-2-penten-4-ynyl)oxime oxalate, mp 106-110°C.

### EXAMPLE 15

### Z-(±)-1-Azabicyclo[2.2.1]heptan-3-one, O-(E-3-methyl-5-phenyl-2-penten-4-ynyl)oxime oxalate, mp 133-134°C.

### EXAMPLE 16

### E-(±)-1-Azabicyclo[2.2.1]heptan-3-one, O-(E-3-methyl-5-phenyl-2-penten-4-ynyl)oxime oxalate, mp 129-131°C.

### PREPARATION OF HYDROXYLAMINES

### I. O-(Trans-3-methyl-5-phenyl-2-penten-4-ynyl)-hydroxylamine hydrochloride

(a) Trans-3-methyl-5-phenyl-2-penten-4-yn-1-ol
   Trans-3-methyl-2-penten-4-yn-1-ol (10.57 g, 0.11 mol), diethylamine (80 mL), copper(I)iodide (0.7 g), tetrahydrofuran (30 mL), and tetrakis (triphenylphosphine)palladium(o) (1.0 g) were placed in a 300-mL flask under nitrogen. Iodobenzene (11.2 mL, 0.1 mol) was added dropwise via a dropping funnel at room temperature. After stirring at room temperature for 24 hours, the reaction was evaporated in vacuo to give a brown semisolid residue. The crude residue was dissolved in 200 mL of water, then extracted four times with 200 mL of diethyl ether. The combined extracts were dried over anhydrous sodium sulfate and evaporated in vacuo to give a clear brown liquid. This was purified by column chromatography (silica gel), eluting with 2:1 hexane-ethyl acetate to afford 12.5 g of the title product.
   ¹H NMR: δ (CDCl₃) 7.2-7.45 (5H, m), 6.06-6.13 (1H, m), 4.25-4.28 (2H, d), 1.92 (3H, s), 1.76 (1H, br.s). ¹³C NMR: δ (CDCl₃) 17.62, 59.2, 60.5, 87.7, 91.5, 120.85, 123.2, 128.1, 128.3, 131.56, 135.59.
(b) N-(trans-3-methyl-5-phenyl-2-penten-4-ynyl)-oxyphthalimide
   Diethyl azodicarboxylate (15.2 mL, 76 mmol) was added dropwise to a solution of N-hydroxyphthalimide (11.2 g, 69 mmol), trans-3-methyl-5-phenyl-2-penten-4-yn-1-ol (11.83 g, 69 mmol), and triphenylphosphine (18.03 g, 69 mmol) in 500 mL of tetrahydrofuran at room temperature. After stirring at room temperature for 16 hours, the reaction mixture was evaporated in vacuo to give a yellow solid. The solid was purified by column chromatography (silica gel), eluting with dichloromethane to give a yellow solid that was recrystallized from ethanol to 12.29 g of the title product, mp 96-98°C.
(c) O-(Trans-3-methyl-5-phenyl-2-penten-4-ynyl)hydroxylamine hydrochloride
   Methylhydrazine (1 mL, 18.8 mmol) was added dropwise to a solution of N-(trans-3-methyl-5-phenyl-2-penten-4-ynyl)oxyphthalimide (5.96 g, 18.8 mmol) in 20 mL of dichloromethane. A precipitate formed immediately. The reaction was stirred for 2.5 hours, then filtered. The filtrate was diluted to 500 mL with diethyl ether and anhydrous hydrogen chloride gas was bubbled into the dilute solution to give a precipitate of the title product, 4.04 g, mp 196-197°C.

### II. O-(3-(4[-Methoxyphenyl)-2-propynyl)hydroxylamine hydrochloride

(a) 3-(4'-Methoxyphenyl)-2-propyn-1-ol
   Diethylamine (80 mL, propargyl alcohol (6.4 mL, 0.11 mol), copper(I)iodide (0.7 g), tetrahydrofuran (25 mL), and tetrakis(triphenylphosphine)palladium(o) (1.2 g) were placed in a 250-mL flask under nitrogen. A solution of p-iodoanisole (23.4 g, 0.1 mol) in 40 mL of tetrahydrofuran was added to the reaction dropwise over 30 minutes. The reaction was stirred for 16 hours at room temperature, then evaporated in vacuo to give a dark brown residue. The residue was dissolved in 200 mL of water and extracted four times with diethyl ether (200 mL). The combined extracts were dried over anhydrous sodium sulfate and evaporated to give a dark brown solid that was purified by column chromatography (silica gel), eluting with 9:1 hexane-ethyl acetate to give 15.38 g of the title product.
   ¹H NMR: δ (CDCL₃) 7.2-7.3 (2H, d), 6.6-6.7 (2H, d), 4.25-4.40 (2H, d), 3.65 (3H, s), 1.85-2.05 (1H, t).
(b) N-(3-(4'-Methoxyphenyl)-2-propynyl)-oxyphthalimide
   Diethyl azodicarboxylate (21 mL, 104 mmol) was added dropwise to a solution of 3-(4'-methoxyphenyl)-2-propyn-1-ol (15.38 g, 94.8 mmol), N-hydroxyphthalimide (15.47 g, 94.8 mmol), and triphenylphosphine (24.9 g, 94.8 mmol), and in 500 mL of tetrahydrofuran at room temperature under nitrogen. The reaction was stirred for 24 hours, then evaporated in vacuo to give a crude solid. The crude solid was purified by column chromatography (silica gel), eluting with chloroform to give after evaporation of solvent a crystalline solid that was recrystallized from ethanol to give 20.13 g, mp 145-147°C.
(c) O-(3-(4'-Methoxyphenyl)-2-propynyl)hydroxylamine hydrochloride
   Methyl hydrazine (2 mL, 37.6 mmol) was added dropwise to a solution of N-(3-(4'-methoxyphenyl)-2-propynyl)oxyphthalmide (11.55 g, 37.6 mmol) in 40 mL of dichloromethane at 0°C. The reaction was warmed to room temperature and stirred for 3 hours. The reaction was filtered and the filtrate was diluted to 500 mL with diethyl ether. Anhydrous hydrogen chloride gas was bubbled into the dilute solution to give a precipitate of the hydrochloride salt of the title product, 8.21 g, mp 140-142°C.

The compounds of the present invention are centrally acting muscarinic agents and are thus useful as analgesic agents for the treatment of pain in mammals including man, as sleep aids, and as agents for treating the symptoms of senile dementia, Alzheimer's disease, Huntington's chorea, tardive dyskinesia, hyperkinesia, mania, or similar conditions of cerebral insufficiency characterized by decreased cerebral acetylcholine production or release.

The biological activity of compounds of the present invention was evaluated using a number of tests. The activity of compounds of this invention as central muscarinic binding site agonists and antagonists was measured. In the RQNB screening assay, which is described more fully by Mark Watson, et al, J. Pharmacol. Exp. Ther., 1986;237(2):411), rat cerebral cortex tissue was treated with radiolabeled quinuclidinyl benzilate, a known muscarinic binding site antagonist. The concentrations of test compound required to inhibit 50% of the binding of this muscarinic antagonist were then determined.

Similarly, in the RCMD screening assay, described more fully by T. W. Vickroy, et al, 1984;229(3):747), rat cerebral cortex tissue was treated with radiolabeled cis-methyldioxolane, a known muscarinic binding site agonist. The concentrations of test compounds required to inhibit 50% of the binding of this muscarinic agonist were then determined. These values are reported as IC₅₀ concentrations in Table 2 and demonstrate that the compounds of the present invention possess significant muscarinic activity.

**TABLE I**

| Example | IC₅₀ nM RCMD | IC₅₀ nM RQNB |
|---|---|---|
| 1 | 218 | 17658 |
| 3 | 110 | 13101 |
| 5 | 27 | 6927 |
| 7 | 12 | 1305 |
| 9 | 19 | 3882 |
| 11 | 53 | 6300 |
| 12 | 30 | 6092 |
| 13 | 0.15 | 44 |
| 15 | 12 | 1587 |

Similarly, in a RQNB binding assay in CHO-hm1 and CHO-hm2 cells (see T. Bonner, Trends Pharmacol. Sci. 1989 (Suppl. on Subtypes of Muscarinic Receptors IV):11-15), the following data were obtained which further demonstrates the selectivity of certain compounds for the M1 receptor.

**TABLE II**

| Example | RQNB3m2 IC₅₀ nM | RQNB3m1 IC₅₀ nM | RQNB3m2/ RQNB3m1 |
|---|---|---|---|
| 1 | 69300 | 16600 | 4.2 |
| 3 | 84400 | 10600 | 6.80 |
| 5 | 52000 | 7680 | 7.96 |
| 7 | 4020 | 1120 | 3.6 |
| 9 | 20700 | 3340 | 6.2 |
| 11 | 24750 | 4010 | 6.2 |
| 12 | 27200 | 5500 | 5.0 |
| 13 | 120 | 35 | 3.43 |
| 15 | 7510 | 870 | 8.69 |

The following demonstrates the superiority of the selected compounds of the present invention as compared to compounds described in European Patent Publication 0445731.

RQNB/RCMD, RQNB3m2/RQNB3m1 (m₂/m₁), and phosphatidylinositol (PI) turnover data on Examples 3, 5, 13-16, 56 (EP-0445,731A1), and 22 (EP-0445,731A1) is given in Tables III and IV. RQNB3m2/RQNB3m1 and phosphatidylinositol (PI) turnover data was determined in CHO cells selectively expressing human m1, m2, m3, and m5 receptors (Schwarz RD, Boyd DK, Spencer CJ, Woodward, Abstracts of papers, 21st Annual Meeting of the Society for Neuroscience, New Orleans, LA; Society for Neuroscience; Washington, DC, 1991). Large RQNB/RCMD ratios have been shown to be predictive of good muscarinic agonist activity (Tecle H, Bergmeier S, Moos W, Hershenson F, Coughenour L, Davis R, Schwarz R, Moreland D, Dudley D, Abstracts of papers, 197th National Meeting of the American Chemical Society, Dallas, TX; American Chemical Society; Washington, DC, 1989; Abstract MEDI-47). A ratio of RQNB3m2/RQNB3m1 greater than 1 is predictive of selective m1 muscarinic agonist activity. The second messenger for muscarinic subtypes m1, m3, and m5 is phosphatidylinositol (PI). Examples 3, 5, and 56 (EP-0445,731A1) increase PI turnover only in the m1 subtype muscarinic cells (Table III). The compounds showed no inhibition of forsoklin stimulated accumulation of cAMP. cAMP is the second messenger associated with m2 subtype muscarinic cells. These data clearly show that the compounds are selective for the m1 muscarinic subtype. In addition, Examples 3, 5, and 13-16 show superior ml selectivity as indicated by the RQNB3m2/RQNB3m1 ratios (Table III and Table IV).

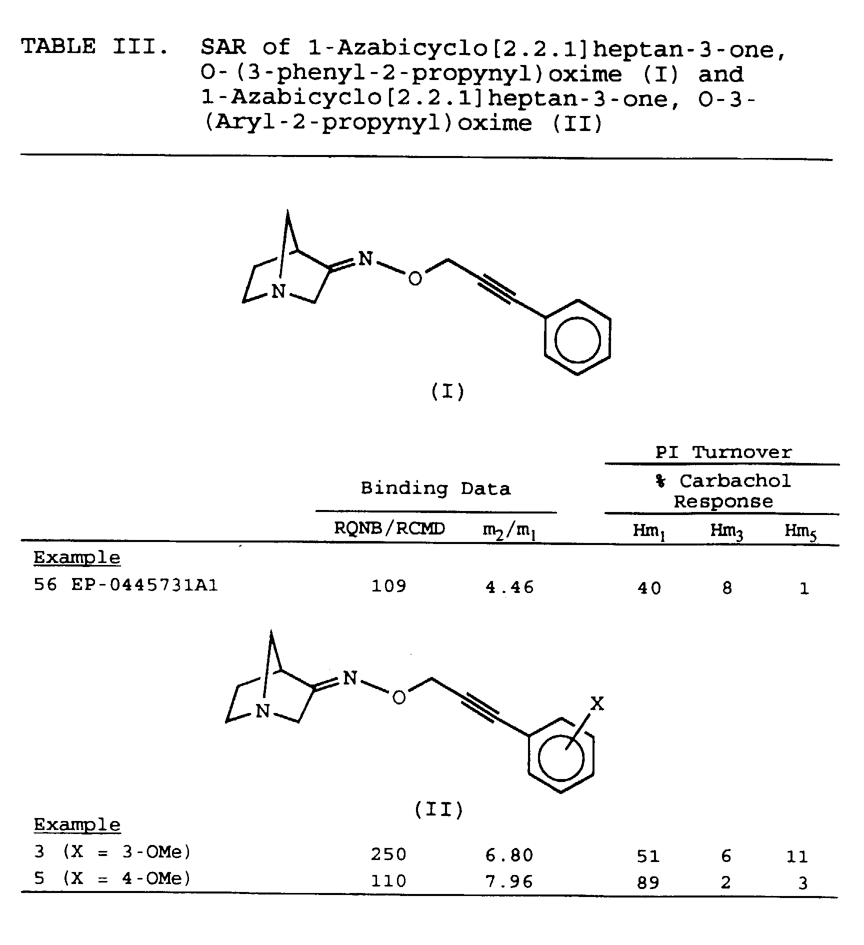

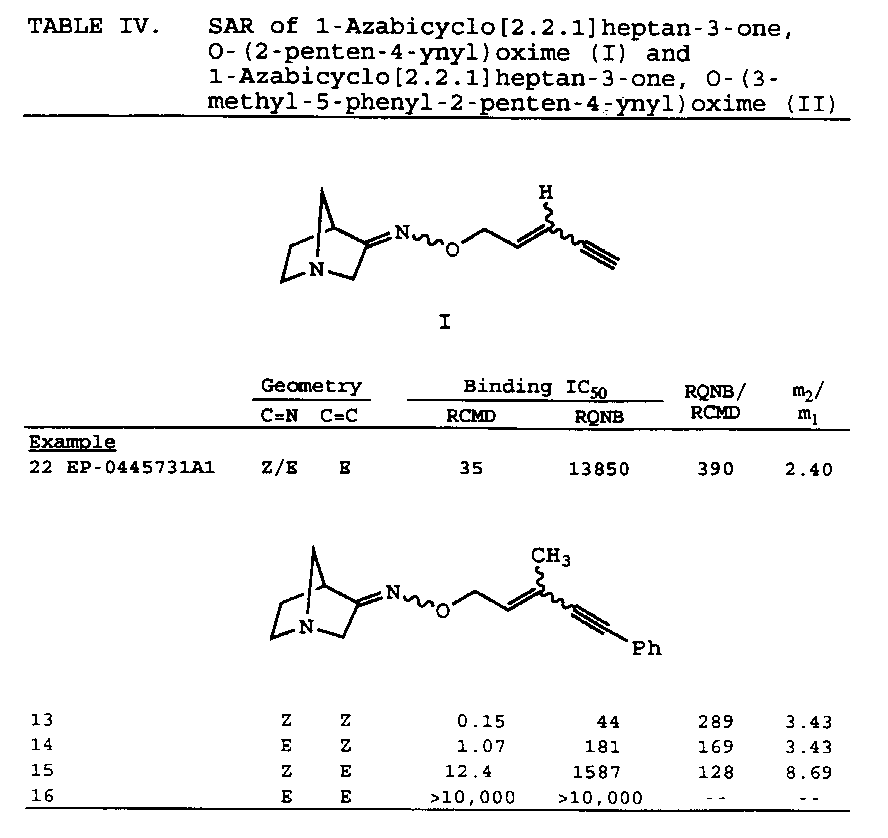

In therapeutic use as agents for treating pain or for treating cerebral insufficiency, the compounds utilized in the pharmaceutical method of this invention are administered to the patient at dosage levels of from 0.07 to 700 mg per day. For a normal human adult of approximately 70 kg of body weight, this translates into a dosage of from 0.01 to 100 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

For preparing suppositories, a low-melting wax such as a mixture of fatty acid glycerides and cocoa butter is first melted, and the active ingredient is dispersed therein by, for example, stirring. The molten homogeneous mixture is then poured into convenient sized molds and allowed to cool and solidify.

Powders and tablets preferably contain between about 5% to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral or parenteral administration, or suspensions, and emulsions suitable for oral administration. Sterile water solutions of the active component or sterile solutions of the active component in solvents comprising water, ethanol, or propylene glycol may be mentioned as examples of liquid preparations suitable for parenteral administration.

Sterile solutions may be prepared by dissolving the active component in the desired solvent system, and then passing the resulting solution through a membrane filter to sterilize it or, alternatively, by dissolving the sterile compound in a previously sterilized solvent under sterile conditions.

Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can a packaged preparation, the package containing discrete quantities of the preparation, for example, packeted tablets, capsules, and powders in vials or ampoules. The unit dosage form can also be capsule, cachet, or tablet itself, or it can be the appropriate number of any of these packaged forms.

## Claims

1. A compound of the formula 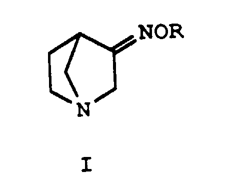 wherein R is selected from the group 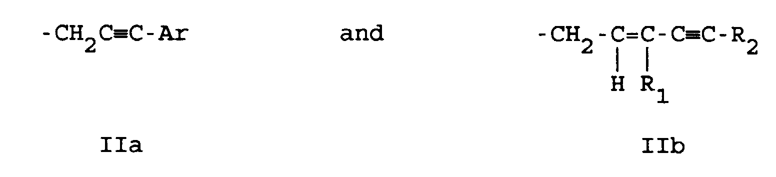 wherein Ar is an aromatic group selected from phenoxy, phenyl, 2- or 3-thienyl, 2- or 3-furanyl, 2- or 3-pyrrolyl, 2-, 3-, or 4-pyridinyl, wherein the aromatic group is substituted with 1 or 2 substituents selected from straight or branched alkyl having from 1 to 4 carbon atoms, straight or branched alkoxy having from 1 to 4 carbon atoms, chlorine, fluorine, bromine, trifluoromethyl, nitro, hydroxy, trifluoromethoxy, amino, amino substituted with 1 or 2 straight or branched lower alkyl groups having from 1 to 4 carbon atoms;
R₁ is an alkyl group of 1 to 4 carbon atoms;
R₂ is hydrogen or Ar or a corresponding unsubstituted aromatic group as defined above; or an individual geometric and optically active isomer, or a pharmaceutically acceptable salt thereof.

2. A compound of Claim 1 wherein Ar is phenyl substituted with one substituent selected from straight alkyl from 1 to 4 carbons, straight alkoxy having from 1 to 4 carbon atoms, chlorine, bromine, trifluoromethyl, or trifluoromethoxy.

3. A compound of Claim 1 wherein R₂ is hydrogen, phenyl or phenyl substituted with one substituent selected from straight alkyl from 1 to 4 carbons, straight alkoxy having from 1 to 4 carbon atoms, chlorine, bromine, trifluoromethyl, or trifluoromethoxy.

4. A compound of Claim 1 wherein R₁ is a methyl group.

5. A compound of Claim 2 which is selected from
Z-(±)-1-Azabicyclo[2.2.1]heptan-3-one, O-(3-(2'-methoxyphenyl)-2-propynyl)oxime;
Z-(+)-1-Azabicyclo[2.2.1]heptan-3-one, O-(3-(2'-methoxyphenyl)-2-propynyl)oxime;
Z-(-)-1-Azabicyclo[2.2.1]heptan-3-one, O-(3-(2'-methoxyphenyl)-2-propynyl)oxime;
Z-(±)-1-Azabicyclo[2.2.1]heptan-3-one, O-(3-(3'-methoxyphenyl)-2-propynyl)oxime;
Z-(+)-1-Azabicyclo[2.2.1]heptan-3-one, O-(3-(3'-methoxyphenyl)-2-propynyl)oxime;
Z-(-)-1-Azabicyclo[2.2.1]heptan-3-one, O-(3-(3'-methoxyphenyl)-2-propynyl)oxime;
Z-(±)-1-Azabicyclo[2.2.1]heptan-3-one, O-(3-(4'-methoxyphenyl)-2-propynyl)oxime;
Z-(+)-1-Azabicyclo[2.2.1]heptan-3-one, O-(3-(4'-methoxyphenyl)-2-propynyl)oxime;
Z-(-)-1-Azabicyclo[2.2.1]heptan-3-one, O-(3-(4'-methoxyphenyl)-2-propynyl)oxime;
Z-(±)-1-Azabicyclo[2.2.1]heptan-3-one, O-(3-(2'-chlorophenyl)-2-propynyl)oxime;
Z-(+)-1-Azabicyclo[2.2.1]heptan-3-one, O-(3-(2'-chlorophenyl)-2-propynyl)oxime;
Z-(-)-1-Azabicyclo[2.2.1]heptan-3-one, O-(3-(2'-chlorophenyl)-2-propynyl)oxime;
Z-(±)-1-Azabicyclo[2.2.1]heptan-3-one, O-(3-(3'-chlorophenyl)-2-propynyl)oxime;
Z-(+)-1-Azabicyclo[2.2.1]heptan-3-one, O-(3-(3'-chlorophenyl)-2-propynyl)oxime;
Z-(-)-1-Azabicyclo[2.2.1]heptan-3-one, O-(3-(3'-chlorophenyl)-2-propynyl)oxime;
Z-(±)-1-Azabicyclo[2.2.1]heptan-3-one, O-(3-(4'-chlorophenyl)-2-propynyl)oxime;
Z-(+)-1-Azabicyclo[2.2.1]heptan-3-one, O-(3-(4'-chlorophenyl)-2-propynyl)oxime;
Z-(-)-1-Azabicyclo[2.2.1]heptan-3-one, O-(3-(4'-chlorophenyl)-2-propynyl)oxime;
Z-(±)-1-Azabicyclo[2.2.1]heptan-3-one, O-(3-(2'-fluorophenyl)-2-propynyl)oxime;
Z-(+)-1-Azabicyclo[2.2.1]heptan-3-one, O-(3-(2'-fluorophenyl)-2-propynyl)oxime;
Z-(-)-1-Azabicyclo[2.2.1]heptan-3-one, O-(3-(2'-fluorophenyl)-2-propynyl)oxime;
Z-(±)-1-Azabicyclo[2.2.1]heptan-3-one, O-(3-(3'-fluorophenyl)-2-propynyl)oxime;
Z-(+)-1-Azabicyclo[2.2.1]heptan-3-one, O-(3-(3'-fluorophenyl)-2-propynyl)oxime;
Z-(-)-1-Azabicyclo[2.2.1]heptan-3-one, O-(3-(3'-fluorophenyl)-2-propynyl)oxime;
Z-(±)-1-Azabicyclo[2.2.1]heptan-3-one, O-(3-(4'-fluorophenyl)-2-propynyl)oxime;
Z-(+)-1-Azabicyclo[2.2.1]heptan-3-one, O-(3-(4'-fluorophenyl)-2-propynyl)oxime;and
Z-(-)-1-Azabicyclo[2.2.1]heptan-3-one, O-(3-(4'-fluorophenyl)-2-propynyl)oxime.

6. A compound according to Claim 3, which is selected from
Z-(±)-1-Azabicyclo[2.2.1]heptan-3-one, O-(Z-3-methyl-5-phenyl-2-penten-4-ynyl)oxime;
Z-(+)-1-Azabicyclo[2.2.1]heptan-3-one, O-(Z-3-methyl-5-phenyl-2-penten-4-ynyl)oxime;
Z-(-)-1-Azabicyclo[2.2.1]heptan-3-one, O-(Z-3-methyl-5-phenyl-2-penten-4-ynyl)oxime;
Z-(±)-1-Azabicyclo[2.2.1]heptan-3-one, O-(E-3-methyl-5-phenyl-2-penten-4-ynyl)oxime;
Z-(+)-1-Azabicyclo[2.2.1]heptan-3-one, O-(E-3-methyl-5-phenyl-2-penten-4-ynyl)oxime;
Z-(-)-1-Azabicyclo[2.2.1]heptan-3-one, O-(E-3-methyl-5-phenyl-2-penten-4-ynyl)oxime;
E-(±)-1-Azabicyclo[2.2.1]heptan-3-one, O-(Z-3-methyl-5-phenyl-2-penten-4-ynyl)oxime;
E-(+)-1-Azabicyclo[2.2.1]heptan-3-one, O-(Z-3-methyl-5-phenyl-2-penten-4-ynyl)oxime;
E-(-)-1-Azabicyclo[2.2.1]heptan-3-one, O-(Z-3-methyl-5-phenyl-2-penten-4-ynyl)oxime;
E-(±)-1-Azabicyclo[2.2.1]heptan-3-one, O-(E-3-methyl-5-phenyl-2-penten-4-ynyl)oxime;
E-(+)-1-Azabicyclo[2.2.1]heptan-3-one, O-(E-3-methyl-5-phenyl-2-penten-4-ynyl)oxime;
E-(-)-1-Azabicyclo[2.2.1]heptan-3-one, O-(E-3-methyl-5-phenyl-2-penten-4-ynyl)oxime;
Z-(±)-1-Azabicyclo-[2.2.1]heptan-3-one, O-(Z-3-methyl-2-penten-4-ynyl)oxime;
Z-(+)-1-Azabicyclo-[2.2.1]heptan-3-one, O-(Z-3-methyl-2-penten-4-ynyl)oxime;
Z-(-)-1-Azabicyclo-[2.2.1]heptan-3-one, O-(Z-3-methyl-2-penten-4-ynyl)oxime;
Z-(±)-1-Azabicyclo-[2.2.1]heptan-3-one, O-(E-3-methyl-2-penten-4-ynyl)oxime;
Z-(+)-1-Azabicyclo-[2.2.1]heptan-3-one, O-(E-3-methyl-2-penten-4-ynyl)oxime;
Z-(-)-1-Azabicyclo-[2.2.1]heptan-3-one, O-(E-3-methyl-2-penten-4-ynyl)oxime;
E-(±)-1-Azabicyclo-[2.2.1]heptan-3-one, O-(Z-3-methyl-2-penten-4-ynyl)oxime;
E-(+)-1-Azabicyclo-[2.2.1]heptan-3-one, O-(Z-3-methyl-2-penten-4-ynyl)oxime;
E-(-)-1-Azabicyclo-[2.2.1]heptan-3-one, O-(Z-3-methyl-2-penten-4-ynyl)oxime;
E-(±)-1-Azabicyclo-[2.2.1]heptan-3-one, O-(E-3-methyl-2-penten-4-ynyl)oxime;
E-(+)-1-Azabicyclo-[2.2.1]heptan-3-one, O-(E-3-methyl-2-penten-4-ynyl)oxime; and
E-(-)-1-Azabicyclo-[2.2.1]heptan-3-one, O-(E-3-methyl-2-penten-4-ynyl)oxime.

7. A pharmaceutically acceptable salt of a compound of Claim 1 wherein the salt is a hydrochloride or an oxalate.

8. A pharmaceutical composition comprising a compound of Claims 1 to 6 together with a pharmaceutically acceptable carrier.

9. Use of a compound of claims 1 to 6 for the preparation of a pharmaceutical useful for alleviating pain in a mammal.

10. Use of a compound of claims 1 to 6 for the preparation of a pharmaceutical useful for treating the symptoms of cognitive decline in a patient in need thereof.

## Patentansprüche

1. Verbindung der Formel: 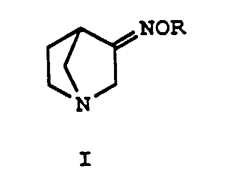 worin R aus der Gruppe: 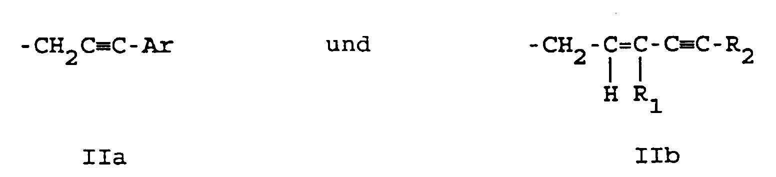 mit Ar gleich einer aromatischen Gruppe, ausgewählt aus Phenoxy, Phenyl, 2- oder 3-Thienyl, 2- oder 3-Furanyl, 2- oder 3-Pyrrolyl, 2-, 3- oder 4-Pyridinyl, wobei die aromatische Gruppe durch 1 oder 2 Substituenten, ausgewählt aus gerad- oder verzweigtkettigem Alkyl mit 1 bis 4 Kohlenstoffatom(en), gerad- oder verzweigtkettigem Alkoxy mit 1 bis 4 Kohlenstoffatom(en), Chlor, Fluor, Brom, Trifluormethyl, Nitro, Hydroxy, Trifluormethoxy, Amino oder durch 1 oder 2 gerad- oder verzweigtkettige Niedrigalkylgruppe(n) mit 1 bis 4 Kohlenstoffatom(en) substituiertes Amino, substituiert ist;
R₁ gleich einr Alkylgruppe mit 1 bis 4 Kohlenstoffatom(en) und
R₂ gleich Wasserstoff oder Ar oder einer entsprechenden unsubstituierten aromatischen Gruppe gemäß obiger Definition,
ausgewählt ist,
oder ein einzelnes geometrisches und optisch aktives Isomer oder ein pharmazeutisch akzeptables Salz hiervon.

2. Verbindung nach Anspruch 1, wobei Ar für durch einen Substituenten, ausgewählt aus geradkettigem Alkyl mit 1 bis 4 Kohlenstoffatom(en), geradkettigem Alkoxy mit 1 bis 4 Kohlenstoffatom(en), Chlor, Brom, Trifluormethyl oder Trifluormethoxy, substituiertes Phenyl steht.

3. Verbindung nach Anspruch 1, wobei R₂ für Wasserstoff, Phenyl oder durch einen Substituenten, ausgewählt aus geradkettigem Alkyl mit 1 bis 4 Kohlenstoffatom(en), geradkettigem Alkoxy mit 1 bis 4 Kohlenstoffatom(en), Chlor, Brom, Trifluormethyl oder Trifluormethoxy, substituiertes Phenyl steht.

4. Verbindung nach Anspruch 1, wobei R₁ für eine Methylgruppe steht.

5. Verbindung nach Anspruch 2, ausgewählt aus
Z-(±)-1-Azabicyclo[2.2.1]heptan-3-on, O-(3-(2'-Methoxyphenyl)-2-propinyl)oxim;
Z-(+)-1-Azabicyclo[2.2.1]heptan-3-on, O-(3-(2'-Methoxyphenyl)-2-propinyl)oxim;
Z-(-)-1-Azabicyclo[2.2.1]heptan-3-on, O-(3-(2'-Methoxyphenyl)-2-propinyl)oxim;
Z-(±)-1-Azabicyclo[2.2.1]heptan-3-on, O-(3-(3'-Methoxyphenyl)-2-propinyl)oxim;
Z-(+)-1-Azabicyclo[2.2.1]heptan-3-on, O-(3-(3'-Methoxyphenyl)-2-propinyl)oxim;
Z-(-)-1-Azabicyclo[2.2.1]heptan-3-on, O-(3-(3'-Methoxyphenyl)-2-propinyl)oxim;
Z-(±)-1-Azabicyclo[2.2.1]heptan-3-on, O-(3-(4'-Methoxyphenyl)-2-propinyl)oxim;
Z-(+)-1-Azabicyclo[2.2.1]heptan-3-on, O-(3-(4'-Methoxyphenyl)-2-propinyl)oxim;
Z-(-)-1-Azabicyclo[2.2.1]heptan-3-on, O-(3-(4'-Methoxyphenyl)-2-propinyl)oxim;
Z-(±)-1-Azabicyclo[2.2.1]heptan-3-on, O-(3-(2'-chlorphenyl)-2-propinyl)oxim;
Z-(+)-1-Azabicyclo[2.2.1]heptan-3-on, O-(3-(2'-Chlorphenyl)-2-propinyl)oxim;
Z-(-)-1-Azabicyclo[2.2.1]heptan-3-on, O-(3-(2'-Chlorphenyl)-2-propinyl)oxim;
Z-(±)-1-Azabicyclo[2.2.1]heptan-3-on, O-(3-(3'-Chlorphenyl)-2-propinyl)oxim;
Z-(+)-1-Azabicyclo[2.2.1]heptan-3-on, O-(3-(3'-Chlorphenyl)-2-propinyl)oxim;
Z-(-)-1-Azabicyclo[2.2.1]heptan-3-on, O-(3-(3'-Chlorphenyl)-2-propinyl)oxim;
Z-(±)-1-Azabicyclo[2,2,1]heptan-3-on, O-(3-(4'-Chlorphenyl)-2-propinyl)oxim;
Z-(+)-1-Azabicyclo[2.2.1]heptan-3-on, O-(3-(4'-Chlorphenyl)-2-propinyl)oxim;
Z-(-)-1-Azabicyclo[2.2.1]heptan-3-on, O-(3-(4'-Chlorphenyl)-2-propinyl)oxim;
Z-(±)-1-Azabicyclo[2.2.1]heptan-3-on, O-(3-(2'-Fluorphenyl)-2-propinyl)oxim;
Z-(+)-1-Azabicyclo[2.2.1]heptan-3-on, O-(3-(2'-Fluorphenyl)-2-propinyl)oxim;
Z-(-)-1-Azabicyclo[2.2.1]heptan-3-on, O-(3-(2'-Fluorphenyl)-2-propinyl)oxim;
Z-(±)-1-Azabicyclo[2.2.1]heptan-3-on, O-(3-(3'-Fluorphenyl)-2-propinyl)oxim;
Z-(+)-1-Azabicyclo[2.2.1]heptan-3-on, O-(3-(3'-Fluorphenyl)-2-propinyl)oxim;
Z-(-)-1-Azabicyclo[2.2.1]heptan-3-on, O-(3-(3'-Fluorphenyl)-2-propinyl)oxim;
Z-(±)-1-Azabicyclo[2.2.1]heptan-3-on, O-(3-(4'-Fluorphenyl)-2-propinyl)oxim;
Z-(+)-1-Azabicyclo[2.2.1]heptan-3-on, O-(3-(4'-Fluorphenyl)-2-propinyl)oxim und
Z-(-)-1-Azabicyclo[2.2.1]heptan-3-on, O-(3-(4'-Fluorphenyl)-2-propinyl)oxim.

6. Verbindung nach Anspruch 3, ausgewählt aus
Z-(±)-1-Azabicyclo[2.2.1]heptan-3-on, O-(Z-3-Methyl-5-phenyl-2-penten-4-inyl)oxim;
Z-(+)-1-Azabicyclo[2.2.1]heptan-3-on, O-(Z-3-Methyl-5-phenyl-2-penten-4-inyl)oxim;
Z-(-)-1-Azabicyclo[2.2.1]heptan-3-on, O-(Z-3-Methyl-5-phenyl-2-penten-4-inyl)oxim;
Z-(±)-1-Azabicyclo[2.2.1]heptan-3-on, O-(E-3-Methyl-5-phenyl-2-penten-4-inyl)oxim;
Z-(+)-1-Azabicyclo[2.2.1]heptan-3-on, O-(E-3-Methyl-5-phenyl-2-penten-4-inyl)oxim;
Z-(-)-1-Azabicyclo[2.2.1]heptan-3-on, O-(E-3-Methyl-5-phenyl-2-penten-4-inyl)oxim;
E-(±)-1-Azabicyclo[2.2.1]heptan-3-on, O-(Z-3-Methyl-5-phenyl-2-penten-4-inyl)oxim;
E-(+)-1-Azabicyclo[2.2.1]heptan-3-on, O-(Z-3-Methyl-5-phenyl-2-penten-4-inyl)oxim;
E-(-)-1-Azabicyclo[2.2.1]heptan-3-on, O-(Z-3-Methyl-5-phenyl-2-penten-4-inyl)oxim;
E-(±)-1-Azabicyclo[2.2.1]heptan-3-on, O-(E-3-Methyl-5-phenyl-2-penten-4-inyl)oxim;
E-(+)-1-Azabicyclo[2.2.1]heptan-3-on, O-(E-3-Methyl-5-phenyl-2-penten-4-inyl)oxim;
E-(-)-1-Azabicyclo[2.2.1]heptan-3-on, O-(E-3-Methyl-5-phenyl-2-penten-4-inyl)oxim;
Z-(±)-1-Azabicyclo[2.2.1]heptan-3-on, O-(Z-3-Methyl-2-penten-4-inyl)oxim;
Z-(+)-1-Azabicyclo[2.2.1]heptan-3-on, O-(Z-3-Methyl-2-penten-4-inyl)oxim;
Z-(-)-1-Azabicyclo[2.2.1]heptan-3-on, O-(Z-3-Methyl-2-penten-4-inyl)oxim;
Z-(±)-1-Azabicyclo[2.2.1]heptan-3-on, O-(E-3-Methyl-2-penten-4-inyl)oxim;
Z-(+)-1-Azabicyclo[2.2.1]heptan-3-on, O-(E-3-Methyl-2-penten-4-inyl)oxim;
Z-(-)-1-Azabicyclo[2.2.1]heptan-3-on, O-(E-3-Methyl-2-penten-4-inyl)oxim;
E-(±)-1-Azabicyclo[2.2.1]heptan-3-on, O-(Z-3-Methyl-2-penten-4-inyl)oxim;
E-(+)-1-Azabicyclo[2.2.1]heptan-3-on, O-(Z-3-Methyl-2-penten-4-inyl)oxim;
E-(-)-1-Azabicyclo[2.2,1]heptan-3-on, O-(Z-3-Methyl-2-penten-4-inyl)oxim;
E-(±)-1-Azabicyclo[2.2.1]heptan-3-on, O-(E-3-Methyl-2-penten-4-inyl)oxim;
E-(+)-1-Azabicyclo[2.2.1]heptan-3-on, O-(E-3-Methyl-2-penten-4-inyl)oxim und
E-(-)-1-Azabicyclo[2.2.1]heptan-3-on, O-(E-3-Methyl-2-penten-4-inyl)oxim.

7. Pharmazeutisch akzeptables Salz einer Verbindung nach Anspruch 1, nämlich ein Hydrochlorid oder ein Oxalat.

8. Arzneimittelzubereitung, umfassend eine Verbindung nach Ansprüchen 1 bis 6 zusammen mit einem pharmazeutisch akzeptablen Träger.

9. Verwendung einer Verbindung nach Ansprüchen 1 bis 6 zur Herstellung eines Arzneimittels zur Schmerzlinderung bei einem Säugetier.

10. Verwendung einer Verbindung nach Ansprüchen 1 bis 6 zur Herstellung eines Arzneimittels zur Behandlung der Symptome von nachlassendem Gedächtnis bei einem Patienten mit Bedarf hierfür.

## Revendications

1. Un composé de formule 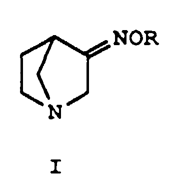 dans laquelle R est choisi parmi le groupe 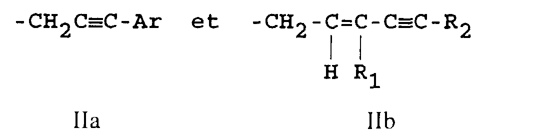 où Ar est un groupe aromatique choisi parmi phénoxy, phényle, 2- ou 3-thiényle, 2- ou 3-furanyle, 2- ou 3-pyrrolyle, 2-, 3-, ou 4-pyridinyle, où le groupe aromatique est substitué par 1 ou 2 substituants choisis parmi les alkyles linéaires ou ramifiés comportant 1 à 4 atomes de carbone, alkoxy linéaires ou ramifiés comportant 1 à 4 atomes de carbone, chlore, fluor, brome, trifluorométhyle, nitro, hydroxy, trifluorométhoxy, amino, amino substitué par 1 ou 2 groupes alkyles inférieurs linéaires ou ramifiés comportant 1 à 4 atomes de carbone;
R₁ est un groupe alkyle comportant 1 à 4 atomes de carbone;
R₂ est un hydrogène ou un groupe aromatique non substitué correspondant tel que défini ci-dessus;
ou bien chacun de leurs isomères géométrique et optiquement actif ou leurs sels pharmaceutiquement acceptable.

2. Un composé selon la revendication 1, dans lequel Ar est un phényle substitué par un substituant choisi parmi les alkyles linéaires comportant 1 à 4 atomes de carbone, alkoxy linéaires comportant 1 à 4 atomes de carbone, chlore, brome, trifluorométhyle, ou trifluorométhoxy.

3. Un composé selon la revendication 1, dans lequel R₂ est un hydrogène, un phényle ou un phényle substitué par un substituant choisi parmi les alkyles linéaires comportant 1 à 4 atomes de carbone, alkoxy linéaires comportant 1 à 4 atomes de carbone, chlore, brome, trifluorométhyle ou trifluorométhoxy.

4. Un procédé selon la revendication 1, dans lequel R1 est groupe méthyl.

5. Un composé selon la revendication 2, choisi parmi:
Z-(±)-1-Azabicyclo[2.2.1]heptan-3-one, O-(3-(2'-méthoxyphényl)-2-propynyl)oxime;
Z-(+)-1-Azabicyclo[2.2.1]heptan-3-one, O-(3-(2'méthoxyphényl)-2-propynyl)oxime;
Z-(-)-1-Azabicyclo[2.2.1]heptan-3-one, O-(3-(2'-méthoxyphényl)-2-propynyl)oxime;
Z-(±)-1-Azabicyclo[2.2.1]heptan-3-one, O-(3-(3'-méthoxyphényl)-2-propynyl)oxime;
Z-(+)-1-Azabicyclo[2.2.1]heptan-3-one, O-(3-(3'-méthoxyphényl)-2-propynyl)oxime;
Z-(-)-1-Azabicyclo[2.2.1]heptan-3-one, O-(3-(3'-méthoxyphényl)-2-propynyl)oxime;
Z-(±)-1-Azabicyclo[2.2.1]heptan-3-one, O-(3-(4'-méthoxyphényl)-2-propynyl)oxime;
Z-(+)-1-Azabicyclo[2.2.1]heptan-3-one, O-(3-(4'-méthoxyphényl)-2-propynyl)oxime;
Z-(-)-1-Azabicyclo[2.2.1]heptan-3-one, O-(3-(4'-méthoxyphényl)-2-propynyl)oxime;
Z-(±)-1-Azabicyclo[2.2.1]heptan-3-one, O-(3-(2'-chlorophényl)-2-propynyl)oxime;
Z-(+)-1-Azabicyclo[2.2.1]heptan-3-one, O-(3-(2'-chlorophényl)-2-propynyl)oxime;
Z-(-)-1-Azabicyclo[2.2.1]heptan-3-one, O-(3-(2'-chlorophényl)-2-propynyl)oxime;
Z-(±)-1-Azabicyclo[2.2.1]heptan-3-one, O-(3-(3'-chlorophényl)-2-propynyl)oxime;
Z-(+)-1-Azabicyclo[2.2.1]heptan-3-one, O-(3-(3'-chloroxyphényl)-2-propynyl)oxime;
Z-(-)-1-Azabicyclo[2.2.1]heptan-3-one, O-(3-(3'-chlorophényl)-2-propynyl)oxime;
Z-(±)-1-Azabicyclo[2.2.1]heptan-3-one, O-(3-(4'-chlorophényl)-2-propynyl)oxime;
Z-(+)-1-Azabicyclo[2.2.1]heptan-3-one, O-(3-(4'-chlorophényl)-2-propynyl)oxime;
Z-(-)-1-Azabicyclo[2.2.1]heptan-3-one, O-(3-(4'-chlorophényl)-2-propynyl)oxime;
Z-(±)-1-Azabicyclo[2.2.1]heptan-3-one, O-(3-(2'fluorophényl)-2-propynyl)oxime;
Z-(+)-1-Azabicyclo[2.2.1]heptan-3-one, O-(3-(2'fluorophényl)-2-propynyl)oxime;
Z-(-)-1-Azabicyclo[2.2.1]heptan-3-one, O-(3-(2'-fluorophényl)-2-propynyl)oxime;
Z-(±)-1-Azabicyclo[2.2.1]heptan-3-one, O-(3-(3'-fluorophényl)-2-propynyl)oxime;
Z-(+)-1-Azabicyclo[2.2.1]heptan-3èone, O-(3-(3'-fluorophényl)-2-propynyl)oxime;
Z-(-)-1-Azabicyclo[2.2.1]heptan-3-one, O-(3-(3'-fluorophényl)-2-propynyl)oxime;
Z-(±)-1-Azabicyclo[2.2.1]heptan-3-one, O-(3-(4'-fluorophényl)-2-propynyl)oxime;
Z-(+)-1-Azabicyclo[2.2.1]heptan-3-one, O-(3-(4'-fluorophényl)-2-propynyl)oxime; et
Z-(-)-1-Azabicyclo[2.2.1]heptan-3-one, O-(3-(4'-fluorophényl)-2-propynyl)oxime

6. Un composé selon la revendication 3, choisi parmi:
Z-(±)-1-Azabicyclo[2.2.1]heptan-3-one, O-(Z-3-méthyl-5-phényl-2-penten-4-ynyl)oxime;
Z-(+)-1-Azabicyclo[2.2.1]heptan-3-one, O-(Z-3-méthyl-5-phényl-2-penten-4-ynyl)oxime;
Z-(-)-1-Azabicyclo[2.2.1]heptan-3-one, O-(Z-3-méthyl-5-phényl-2-penten-4-ynyl)oxime;
Z-(±)-1-Azabicyclo[2.2.1]heptan-3-one, O-(E-3-méthyl-5-phényl-2-penten-4-ynyl)oxime;
Z-(+)-1-Azabicyclo[2.2.1]heptan-3-one, O-(E-3-méthyl-5-phényl-2-penten-4-ynyl)oxime;
Z-(-)-1-Azabicyclo[2.2.1]heptan-3-one, O-(E-3-méthyl-5-phényl-2-penten-4-ynyl)oxime;
E-(±)-1-Azabicyclo[2.2.1]heptan-3-one, O-(Z-3-méthyl-5-phényl-2-penten-4-ynyl)oxime;
E-(+)-1-Azabicyclo[2.2.1]heptan-3-one, O-(Z-3-méthyl-5-phényl-2-penten-4-ynyl)oxime;
E-(-)-1-Azabicyclo[2.2.1]heptan-3-one, O-(Z-3-méthyl-5-phényl-2-penten-4-ynyl)oxime;
E-(±)-1-Azabicyclo[2.2.1]heptan-3-one, O-(E-3-méthyl-5-phényl-2-penten-4-ynyl)oxime;
E-(+)-1-Azabicyclo[2.2.1]heptan-3-one, O-(E-3-méthyl-5-phényl-2-penten-4-ynyl)oxime;
E-(-)-1-Azabicyclo[2.2.1]heptan-3-one, O-(E-3-méthyl-5-phényl-2-penten-4-ynyl)oxime;
Z-(±)-1-Azabicyclo[2.2.1]heptan-3-one, O-(Z-3-méthyl-2-penten-4-ynyl)oxime;
Z-(+)-1-Azabicyclo[2.2.1]heptan-3-one, O-(Z-3-méthyl-2-penten-4-ynyl)oxime;
Z-(-)-1-Azabicyclo[2.2.1]heptan-3-one, O-(Z-3-méthyl-2-penten-4-ynyl)oxime;
Z-(±)-1-Azabicyclo[2.2.1]heptan-3-one, O-(E-3-méthyl-2-penten-4-ynyl)oxime;
Z-(+)-1-Azabicyclo[2.2.1]heptan-3-one, O-(E-3-méthyl-2-penten-4-ynyl)oxime;
Z-(-)-1-Azabicyclo[2.2.1]heptan-3-one, O-(E-3-méthyl-2-penten-4-ynyl)oxime;
E-(±)-1-Azabicyclo[2.2.1]heptan-3-one, O-(Z-3-méthyl-2-penten-4-ynyl)oxime;
E-(+)-1-Azabicyclo[2.2.1]heptan-3-one, O-(Z-3-méthyl-2-penten-4-ynyl)oxime;
E-(-)-1-Azabicyclo[2.2.1]heptan-3-one, O-(Z-3-méthyl-2-penten-4-ynyl)oxime;
E-(±)-1-Azabicyclo[2.2.1]heptan-3-one, O-(E-3-méthyl-2-penten-4-ynyl)oxime;
E-(+)-1-Azabicyclo[2.2.1]heptan-3-one, O-(E-3-méthyl-2-penten-4-ynyl)oxime;et
E-(-)-1-Azabicyclo[2.2.1]heptan-3-one, O-(E-3-méthyl-2-penten-4-ynyl)oxime;

7. Un sel pharmaceutiquement acceptable d'un composé selon la revendication 1, dans lequel le sel est un chlorhydrate ou un oxalate.

8. Une composition pharmaceutique comprenant un composé selon une quelconque des revendications 1 à 6, associée à un support pharmaceutiquement acceptable.

9. Utilisation d'un composé selon l'une quelconque des revendications 1 à 6, pour la préparation d'un produit pharmaceutique utile pour soulager la douleur chez un mammifère.

10. Utilisation d'un composé selon l'une quelconque des revendications 1 à 6, pour la préparation d'un produit pharmaceutique utile pour le traitement des symptômes du déclin cognitif chez un patient nécessitant ce traitement.
